# FASCICULE DE BREVET EUROPEEN

(11) **EP 3 920 192 B1**
(45) Date de publication et mention de la délivrance du brevet: **04.06.2025**
(21) Numéro de dépôt: 21187470.6
(22) Date de dépôt: 13.12.2017
(51) Int. Cl.: G21F 7/02, G21F 3/00, E05D 15/10, E05F 15/657, A61B 6/10

(54) **PARAVENT DE RADIOPROTECTION MOBILE**
MOBILER STRAHLENSCHUTZSCHIRM
SCREEN FOR MOBILE RADIATION PROTECTION

(30) Priorité: 14.12.2016 FR 1662400
(43) Date de publication de la demande: 08.12.2021
(62) Demande divisionnaire de: 17821990.3
(73) Titulaire: Lemer Pax, 44240 La Chapelle-sur-Erdre (FR)
(72) Inventeur: LEMER, Pierre-Marie, 44100 NANTES (FR)
(74) Mandataire: Jacobacci Coralis Harle

(56) Documents cités:
- CN-A- 101 916 606
- JP-A- 2001 120 543
- JP-B2- 5 312 955
- US-A1- 2007 252 095
- US-A1- 2008 073 593

## Description

### DOMAINE TECHNIQUE AUQUEL SE RAPPORTE L'INVENTION

La présente invention concerne de manière générale le domaine des équipements de protection contre les rayonnements ionisants.

Elle concerne plus particulièrement les paravents radio-protecteurs qui sont utilisés en milieu médical ou autre, pour protéger un opérateur à l'encontre des émissions de rayonnements ionisants, par exemple les rayons X. Elle concerne également un équipement en forme de housse pour l'habillage par recouvrement de tels paravents, en vue de leur utilisation dans une ambiance protégée stérile.

### ARRIERE-PLAN TECHNOLOGIQUE

Dans le cadre de certains examens ou interventions, on soumet les patients à des rayonnements ionisants, type rayons X notamment, utilisés dans un but de contrôle, de diagnostic ou de traitement.

C'est le cas en particulier pour des interventions du genre cathétérisme, pose de pacemakers, examens vasculaires, neurologiques ou urologiques, CRM (Cardiac Rhythm Management), CRT (Cardiac Resynchronization Therapy) ou encore lors de la mise en œuvre de techniques de fluoroscopie.

En particulier, la fluoroscopie est une technique d'imagerie qui consiste à utiliser les rayons X pour obtenir des images en temps réel d'un objet. Dans le domaine médical, son application permet la visualisation des structures et fonctions des organes internes d'un patient, comme par exemple le battement cardiaque ou le passage du sang dans les vaisseaux sanguins. Cette technique est utilisée pour le diagnostic ainsi que la thérapie ; et elle est présente dans les domaines interventionnels notamment de la radiologie, cardiologie, neurologie, électrophysiologie, radiologie périphérique vasculaire, pédiatrie interventionnelle...

Les salles destinées à ces spécialités sont équipées d'appareils de fluoroscopie (encore appelés C-arm) qui se présentent sous la forme générale d'un caisson technique mobile se prolongeant par un grand arceau dont une extrémité comporte un appareil émetteur de rayon X et dont l'autre extrémité est munie d'un détecteur.

Dans les salles équipées, des cathéters et sondes sont introduits par une voie d'accès (généralement l'artère fémorale ou radiale) dans un but de diagnostic ou thérapeutique. Le réseau vasculaire est visualisé grâce à l'utilisation des rayons X, souvent couplé à une injection de produit(s) de contraste.

Ces appareils de fluoroscopie occupent une place importante autour de la table d'examen et leur positionnement est fréquemment modifié selon la zone corporelle du patient à inspecter ou à traiter.

On comprend qu'il est important de protéger correctement les opérateurs (médecins, chirurgiens, techniciens, infirmières ou autre) contre les rayonnements ionisants émis, (de type primaires, provenant directement de l'émetteur, ou de type secondaires : réfléchis par les équipements et issus du patient lui-même), sous peine de les exposer à des doses importantes, cumulées dans le temps, susceptibles d'engendrer des pathologies diverses (nécroses des membres supérieurs, tumeurs cérébrales, cataractes, radiodermites etc.).

Il existe pour cela des structures de protection consistant en des vêtements du genre blouses, chasubles, tabliers en matériau radio-protecteur, protège-thyroïde, lunettes..., mais qui ne couvrent pas toujours la totalité du corps et dont le poids important nuit au confort de l'opérateur, limite ses capacités de mouvements et entraîne une fatigue rapide.

Il existe encore des écrans ou paravents constitués de panneaux ou d'assemblages de panneaux en matériau radio-protecteur approprié, suspendus à un support adapté ou posés sur le sol, soit directement, soit par l'intermédiaire d'un piètement roulant.

De telles structures de paravents radio-protecteurs sont décrites dans les documents US-2012/0049093, US-2006/0076522, FR-2 915 868, WO-2009/156660, ou encore US-3 308 297.

Cependant, ces différentes structures ne permettent pas à l'opérateur de travailler dans des conditions optimales. En particulier, certaines d'entre elles ne sont pas bien adaptées pour permettre à un opérateur situé du côté protégé du paravent, d'accéder, par ses bras et ses mains, de l'autre côté de ce paravent, par exemple pour intervenir sur une partie du corps du patient exposée aux rayonnements.

En outre, les structures de paravent connues jusqu'à présent s'avèrent souvent gênantes pour le déplacement des équipements de la salle opératoire, notamment les appareils de fluoroscopie.

### OBJET DE L'INVENTION

Afin de remédier aux inconvénients précités de l'état de la technique, la présente invention propose un paravent de radioprotection pour la protection d'au moins un opérateur à l'encontre des rayonnements ionisants, lequel paravent est du type comprenant une structure de paroi avant en matériau(x) radio-protecteur(s) et une structure de paroi de côté en matériau(x) radio-protecteur(s), reliés entre elles au niveau d'une bordure d'angle verticale ou sensiblement verticale, lequel paravent comporte un piètement muni de roues d'appui au sol ;
et conformément à l'invention, ladite structure de paroi avant comprend une partie inférieure et une partie supérieure mobiles l'une par rapport à l'autre, laquelle partie supérieure de ladite structure de paroi avant est montée pivotante au niveau de ladite bordure d'angle, autour d'un axe pivot vertical ou sensiblement vertical.

Avec sa structure de paroi avant positionnée juste devant une table d'examen sur laquelle est allongé un patient, un tel paravent permet de protéger efficacement l'opérateur, tout en lui préservant une grande liberté de mouvements. La partie supérieure mobile pivotante de la structure de paroi avant peut être manœuvrée par l'opérateur sur le côté ou au dessus du patient en fonction des actes à réaliser ; et en cas de contact avec des appareils environnants, par exemple le C-arm de fluoroscopie, cette partie supérieure mobile peut pivoter, évitant ainsi d'avoir à déplacer le paravent dans son ensemble.

D'autres caractéristiques non limitatives et avantageuses du paravent radio-protecteur conforme à l'invention, prises individuellement ou selon toutes les combinaisons techniquement possibles, sont les suivantes :
- la structure de paroi de côté s'étend dans un plan vertical ou sensiblement vertical et comporte : - une bordure latérale libre, - une bordure latérale définissant une partie de ladite bordure d'angle, - une bordure inférieure, et - une bordure supérieure, laquelle structure de paroi de côté comporte une partie supérieure dont au moins une partie est réalisée en matériau radio-protecteur transparent, et une partie inférieure,
   et la partie inférieure de la structure de paroi avant est délimitée par : - une bordure latérale libre, - une bordure latérale définissant une partie de ladite bordure d'angle, - une bordure inférieure, et - une bordure supérieure,
   laquelle partie inférieure de la structure de paroi avant s'étend dans un plan vertical ou sensiblement vertical qui est décalé par rapport au plan de ladite structure de paroi de côté, d'un angle fixe compris entre 70 et 120°, de préférence de l'ordre 90°.
- le paravent comporte une partie réalisée en matériau radio-protecteur souple, qui se présente sous la forme d'un panneau s'étendant sur une partie de la hauteur de ladite bordure d'angle, et sur une partie de ladite partie inférieure de la structure de paroi de côté ainsi que sur une partie de ladite partie inférieure de la structure de paroi avant, de part et d'autre de ladite bordure d'angle.

Plus précisément, ce panneau en matériau radio-protecteur souple s'étend avantageusement :
a/ à partir de la bordure inférieure de ladite partie inférieure de la structure de paroi de côté et de la bordure inférieure de ladite partie inférieure de la structure de paroi avant, sur plus de la moitié de la hauteur desdites parties inférieures de la structure de paroi avant et de la structure de paroi de côté, et
*b*/ à partir de ladite bordure d'angle, sur plus de la moitié de la largeur desdites parties inférieures des structures de parois avant et de côté.
   - la structure de paroi de côté comporte une face intérieure orientée vers l'espace de positionnement de l'opérateur, et une face extérieure opposée ; et la bordure supérieure de ladite structure de paroi de côté comporte un prolongement formant toiture, en matériau radio-protecteur, qui s'étend du côté de ladite face intérieure.
   - la partie inférieure de la structure de paroi avant comporte : une face intérieure orientée vers l'espace de positionnement de l'opérateur, une face extérieure opposée, et une bordure supérieure ;
   et ladite bordure supérieure comporte un prolongement de protection réalisé en matériau radio-protecteur, qui s'étend du côté de ladite face extérieure.

Ce prolongement de protection peut se présenter sous la forme d'une tablette qui s'étend dans une position horizontale ou sensiblement horizontale à partir de la bordure supérieure de la partie inférieure de la structure de paroi avant ; elle est avantageusement montée pivotante sur ladite bordure supérieure de la partie inférieure de la structure de paroi avant, pour permettre son relevage à partir de ladite position horizontale ; et elle comporte avantageusement une extension d'extrémité rétractable pour la rendre télescopique.

Dans une variante de réalisation ce prolongement de protection consiste en une bavette semi-rigide qui s'étend vers le haut à partie de la bordure supérieure de la partie inférieure de la structure de paroi avant, avec une section en arc de cercle ou pratiquement en arc de cercle.
- la partie supérieure de la structure de paroi avant comporte une bordure latérale libre, une bordure latérale formant une partie de ladite bordure d'angle, une bordure supérieure, une bordure inférieure, une face intérieure orientée vers l'espace de positionnement de l'opérateur, et une face extérieure opposée ; et en outre elle est constituée d'au moins un panneau en matériau radio-protecteur.
- la bordure latérale de la structure de paroi de côté qui forme une partie de ladite bordure d'angle comporte une extension en forme d'aile latérale adaptée pour venir recouvrir la bordure latérale en regard de la partie supérieure de la structure de paroi avant, et l'axe pivot associé, ce recouvrement étant réalisé du côté de la face extérieure de ladite partie supérieure de la structure de paroi avant.
- la bordure supérieure de la partie supérieure de la structure de paroi avant comporte un prolongement formant toiture, réalisé en matériau radio-protecteur, qui s'étend du côté de la face intérieure de ladite partie supérieure de la structure de paroi avant, dans un plan décalé par rapport au plan du prolongement formant toiture de la structure de paroi de côté.
- la bordure inférieure de la partie supérieure de la structure de paroi avant est constituée par un rideau flexible formé d'une juxtaposition d'une pluralité de bandes souples en matériau radio-protecteur.
- la partie supérieure de la structure de paroi avant comprend un panneau supérieur dont au moins une partie est réalisée en matériau radio-protecteur transparent, et un panneau inférieur, dont au moins une partie est réalisée en matériau radio-protecteur transparent, lequel panneau inférieur est mobile en translation verticale par rapport au panneau supérieur, pour réaliser une partie supérieure de structure de paroi avant télescopique, réglable en hauteur.

Ledit panneau inférieur mobile est avantageusement solidaire dudit panneau supérieur par l'intermédiaire d'un système d'équilibrage, par exemple, du type ressort spirale à force constante.
- la partie supérieure de la structure de paroi avant comprend un bras support monté pivotant sur ladite bordure d'angle autour dudit axe pivot, et ledit au moins un panneau constitutif de ladite partie supérieure est monté mobile en translation horizontale sur ledit bras support.

L'invention propose également un équipement en forme de housse destiné à recouvrir au moins une partie de la hauteur d'un paravent tel que défini ci-dessus, cet équipement comprenant :
- une poche souple munie d'une ouverture, laquelle poche souple est adaptée pour venir recouvrir au moins partiellement la partie supérieure de la structure de paroi avant par engagement de la bordure inférieure de cette dernière dans ladite ouverture de ladite poche souple, laquelle poche souple est munie d'au moins une partie transparente et de moyens pour sa fixation sur ladite partie supérieure de la structure de paroi avant, et
- au moins un panneau souple adapté pour venir recouvrir au moins partiellement ladite structure de paroi de côté et la partie inférieure de ladite structure de paroi avant,
   ledit au moins un panneau comportant au moins une partie transparente destinée à venir se positionner en regard de la partie transparente de ladite structure de paroi de côté, et des moyens de fixation sur lesdites structures de paroi de côté et de paroi avant, et
   - en cas de présence d'un prolongement de protection prolongeant la bordure supérieure de la partie inférieure de la structure de paroi avant, une structure souple de recouvrement au moins partiel dudit prolongement de protection, soit solidaire dudit panneau pour le recouvrement de ladite structure de paroi de côté et de la partie inférieure de la structure de paroi avant, soit indépendante de ce panneau, laquelle structure souple est munie de moyens pour sa fixation sur ladite tablette.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 représente un paravent radio-protecteur conforme à l'invention, vu en perspective du côté extérieur (à l'opposé de l'espace de positionnement de l'opérateur), avec la partie supérieure de la structure de paroi avant décalée angulairement par rapport à la partie inférieure de ladite structure de paroi avant ;
- la figure 2 montre le paravent radio-protecteur de la figure 1, vu en perspective du côté intérieur, c'est-à-dire du côté de l'espace de positionnement de l'opérateur ;
- la figure 3 est une vue de dessus du paravent radio-protecteur illustré sur les figures 1 et 2 ;
- la figure 4 est une vue de côté du paravent radio-protecteur illustré sur les figures 1 à 3;
- la figure 5 est une vue en coupe du paravent radio-protecteur selon le plan de coupe 5-5 de la figure 4 ;
- la figure 6 est une vue en coupe du paravent radio-protecteur selon le plan de coupe 6-6 de la figure 4 ;
- la figure 7 est une vue en perspective du paravent radio-protecteur des figure 1 à 6, illustré dans une autre configuration de la partie supérieure de sa structure de paroi avant (à savoir disposée dans le plan de la partie inférieure de ladite structure de paroi avant) ;
- la figure 8 est une vue en perspective de la partie supérieure de la structure de paroi avant du paravent radio-protecteur des figures 1 à 7, représentée isolément ;
- la figure 9 est une vue de face de la partie supérieure de la structure de paroi avant illustrée sur la figure 8 ;
- la figure 10 est une vue en coupe selon le plan de coupe 10-10 de la figure 9 ;
- la figure 11 est une vue en perspective du paravent radio-protecteur des figures 1 à 7, illustré encore dans une autre configuration de la partie supérieure de sa structure de paroi avant, adaptée pour la pose d'un équipement en forme de housse stérile, en vue d'une utilisation en salle d'opération ;
- la figure 12 est une vue de côté du paravent radio-protecteur de la figure 11, illustrant l'équipement en forme de housse posé sur ses différentes parties de réception ;
- la figure 13 est une vue en perspective d'une variante de réalisation du paravent radio-protecteur selon l'invention ;
- la figure 14 est une vue de côté du paravent radio-protecteur de la figure 13.

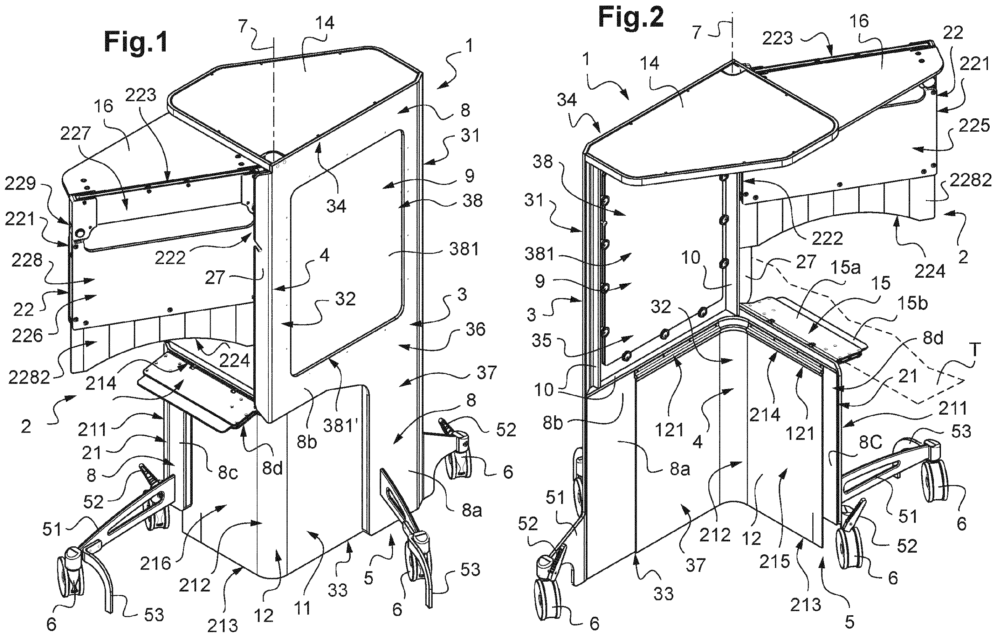

Le paravent de radioprotection 1 illustré sur les figures 1 à 12 est adapté pour assurer la protection d'un opérateur contre les rayonnements ionisants émis par une source de rayonnements ionisants, par exemple des rayons X émis par un appareil de fluoroscopie du type C-arm, dans une salle d'opération hospitalière.

Pour cela les différentes parties constitutives de ce paravent radio-protecteur 1 sont réalisées en matériau(x) radio-protecteur(s) ayant une équivalence plomb adaptée, d'au minimum 0,2 mm, selon les parties constitutives concernées. Par exemple, cette équivalence plomb est comprise entre 0, 5 mm et 3 mm.

Tel qu'illustré sur les figures 1 à 7, ce paravent radio-protecteur 1 se présente sous la forme d'une cabine mobile comprenant une structure de paroi avant 2 en matériau(x) radio-protecteur(s) et une structure de paroi de côté 3 en matériau(x) radio-protecteur(s), reliées entre elles au niveau d'une bordure d'angle 4 verticale ou sensiblement verticale, l'ensemble étant monté sur un piètement 5 muni de roues 6 d'appui au sol.

La structure de paroi de côté 3 se présente sous la forme d'un panneau s'étendant dans un plan vertical ou sensiblement vertical ; et la structure de paroi avant 2 comprend deux parties :
- une partie inférieure 21, fixe par rapport à la structure de paroi de côté 3, qui s'étend dans un plan vertical ou sensiblement vertical, et
- une partie supérieure 22, qui s'étend également dans un plan vertical ou sensiblement vertical, et qui est montée pivotante au niveau de la bordure d'angle 4, au dessus de la partie inférieure 21, autour d'un axe pivot 7 vertical ou sensiblement vertical.

Cette partie supérieure 22 est donc mobile par rapport à la structure de paroi de côté 3; et elle est aussi mobile par rapport à la partie inférieure 21, au-dessus de cette dernière.

La structure de paroi de côté 3 comporte :
- une bordure latérale libre 31,
- une bordure latérale 32 définissant une partie de ladite bordure d'angle 4,
- une bordure inférieure 33,
- une bordure supérieure 34,
- une face intérieure 35 orientée vers l'espace destiné au positionnement de l'opérateur,
- une face extérieure 36, opposée à la face intérieure 35,
- une partie inférieure 37, et
- une partie supérieure 38, dont au moins une partie 381 est réalisée en matériau radio-protecteur transparent.

La partie inférieure 37 et la partie supérieure 38 s'étendent dans le prolongement l'une de l'autre, la bordure inférieure horizontale 381' de la partie transparente 381 constituant par définition la ligne de séparation entre lesdites parties supérieure 38 et inférieure 37 (et constituant ainsi la bordure inférieure de la partie supérieure 38 et la bordure supérieure de la partie inférieure 37). La hauteur de la partie supérieure 38 peut être du même ordre que la hauteur de la partie inférieure 37.

La partie inférieure 21 de la structure de paroi avant 2 est délimitée par : - une bordure latérale libre 211,
- une bordure latérale 212 définissant une partie de la bordure d'angle 4,
- une bordure inférieure 213, et
- une bordure supérieure 214.

Cette partie inférieure 21 de la structure de paroi avant 2 comporte encore une face intérieure 215 orientée vers l'espace destiné au positionnement de l'opérateur, et une face extérieure 216, opposée à la face intérieure 215 ; et cette partie inférieure 21 s'étend dans un plan vertical ou sensiblement vertical qui est décalé par rapport au plan de ladite structure de paroi de côté 3, d'un angle fixe ici de 90°, vu du côté desdites faces intérieures 215 et 35.

Dans des variantes de réalisation cet angle fixe peut être différent, par exemple compris entre 70 et 120°.

La structure de paroi de côté 3 et la partie inférieure 21 de la structure de paroi avant 2 comprennent une ou plusieurs parties en matériau radio-protecteur tel que le plomb, l'acier, la fonte, par exemple, d'une équivalence plomb d'au moins 0,2 mm, constituant un châssis 8 du paravent 1, sur la partie basse duquel sont fixées les roues d'appui au sol 6 formant le piètement 5.

Ces roues 6, au minimum au nombre de 3, ici au nombre de 4, sont fixées sur le châssis 8 par l'intermédiaire de bras 51.

Certaines au moins des roues 6 sont équipées d'un levier de verrouillage 52 pour le verrouillage amovible de leur rotation (par exemple les roues 6 accessibles depuis le côté intérieur du paravent 1) ; et certaines au moins des roues 6 sont ici équipées d'un pied anti basculement 53 (par exemple les roues 6 accessibles depuis le côté extérieur du paravent 1). Dans une variante de réalisation, ces pieds anti basculement 53 peuvent ne pas être présents.

Une partie des roues 6 équipe la partie inférieure 37 de la structure de paroi de côté 3, et une autre partie des roues 6 équipe la partie inférieure 21 de la structure de paroi avant 2.

Une large ouverture centrale 9 est prévue dans la partie de châssis 8 correspondant à la partie supérieure 38 de la structure de paroi de côté 3, pour la réception d'un panneau transparent formant la partie précitée 381 en matériau radio-protecteur transparent. Ce panneau 381 peut être réalisé en plastique plombé, type « Kyowaglass » (marque déposée), plastique radio-protecteur, type « Novashield glass » (marque déposée), verre au plomb, ou autre, le tout d'équivalence plomb comprise entre 0,2 mm et 3 mm. Il est fixé sur le pourtour de l'ouverture 9, du côté de la face intérieure 35, par tout moyen approprié étanche aux rayonnements ionisants. Ce panneau transparent 381 s'étend sur pratiquement toute la largeur et pratiquement toute la hauteur de la partie supérieure 38 de la structure de paroi de côté 3. Il présente une forme rectangulaire de l'ordre de 60 cm de large et 80 cm de haut.

Des profilés de rigidification 10 sont prévus sur au moins une partie du pourtour de l'ouverture centrale 9 pour améliorer les caractéristiques de résistance/rigidité du châssis 8 du paravent radio-protecteur 1.

Sur les figures 1 et 2 on remarque que la partie inférieure du châssis 8 du paravent 1 comporte une large ouverture 11, permettant le passage d'un équipement, tel qu'une partie d'extrémité d'un C-arm de fluoroscopie, cette ouverture 11 étant comblée par un panneau 12 en matériau radio-protecteur souple, capable de se déformer sous le contact dudit équipement tout en constituant une barrière aux rayonnements ionisants.

L'ouverture 11 et donc le panneau 12 en matériau radio-protecteur souple, s'étendent au niveau de la partie inférieure de l'angle du paravent 1, c'est-à-dire qu'ils s'étendent, partant de la bordure d'angle 4 et des bordures inférieures 33 et 213 : sur une partie de la hauteur et de la largeur de la partie inférieure 37 de la structure de paroi de côté 3, ainsi que sur une partie de la hauteur et de la largeur de la partie inférieure 21 de la structure de paroi avant 2.

Plus précisément, l'ouverture 11 et le panneau en matériau radio-protecteur souple 12 s'étendent :
- à partir de la bordure inférieure 33 de la partie inférieure 37 de la structure de paroi de côté 3 et de la bordure inférieure 213 de la partie inférieure 21 de la structure de paroi avant 2 : sur plus de la moitié de la hauteur desdites parties inférieures 37 et 21 de la structure de paroi de côté 3 et de la structure de paroi avant 2, et
- à partir de la bordure d'angle 4 : sur plus de la moitié de la largeur desdites parties inférieures 37 et 21 des structures de parois avant 2 et de côté 3.

Sur la partie inférieure 37 de la structure de paroi de côté 3, le châssis 8 comporte donc une bande latérale 8a sur laquelle sont fixés certains des bras 51 support de roues 6, et une bande supérieure 8b.

De son côté, sur la partie inférieure 21 de la structure de paroi avant 2, le châssis 8 comporte une bande latérale 8c sur laquelle sont fixés certains des bras 51 support de roues 6, et une bande supérieure 8d.

Le panneau 12 en matériau radio-protecteur souple est fixé par sa bordure supérieure 121 sur les bandes supérieures 8b et 8d du châssis 8 (c'est-à-dire à proximité des bordures supérieures 214 et 381' des parties inférieures 21 et 37), du côté des faces intérieures 215 et 35 des structures de parois avant 2 et de côté 3. Les bordures verticales de ce panneau souple 12 sont laissées libres ou fixées avec des moyens élastiques sur le châssis 8, permettant à ce panneau 12 de pouvoir se déformer notamment vers l'intérieur du paravent 1.

Les dimensions du panneau 12 en matériau radio-protecteur souple sont un peu plus grandes que celles de l'ouverture 11 pour permettre un léger recouvrement en partie supérieure et sur les côtés, de manière à assurer la fonction de protection recherchée.

Le panneau 12 peut être constitué d'une ou de plusieurs feuilles de PVC ou de caoutchouc souple, chargées de plomb ou autre matériau radio-protecteur, d'une équivalence plomb d'au minimum 0,20 mm.

A titre de variante, le panneau 12 peut être remplacé par une juxtaposition de bandes verticales en matériau radio-protecteur, par exemple des bandes monobloc en matériau souple, ou des bandes réalisées par assemblage d'éléments rigides reliés deux à deux par une articulation.

Comme on peut le voir sur les figures 1 à 3, la bordure supérieure 34 de la structure de paroi de côté 3 comporte un prolongement formant toiture 14, en matériau radio-protecteur, qui s'étend du côté de sa face intérieure 35.
Ce prolongement 14 s'étend ici à l'équerre par rapport au plan de la structure de paroi de côté 3 et sur toute la largeur de la bordure supérieure 34 ; il est avantageusement réalisé en matériau transparent, par exemple en plastique plombé type « Kyowaglass » (marque déposée), plastique radio-protecteur, type « Novashield glass » (marque déposée), verre au plomb, ou autre, le tout d'équivalence plomb comprise entre 0,2 mm et 3 mm.

La bordure supérieure 214 de la partie inférieure 21 de la structure de paroi avant 2 comporte un prolongement en forme de tablette 15, réalisée en matériau radio-protecteur, qui s'étend du côté de la face extérieure 216.

Cette tablette 15 s'étend dans une position horizontale ou sensiblement horizontale à partir de la bordure supérieure 214, c'est-à-dire perpendiculairement ou sensiblement perpendiculairement au plan vertical de la partie inférieure 21 de la structure de paroi avant 2 ; elle comporte avantageusement une base rigide 15a, qui se prolonge par une extension d'extrémité rigide 15b rétractable, pour la rendre télescopique afin de pouvoir adapter sa longueur.

La tablette 15 est avantageusement montée pivotante sur la bordure supérieure 214 de la partie inférieure 21, pour permettre son relevage à partir de sa position horizontale de stabilité.

Cette possibilité de relevage est intéressante, par exemple pour éviter de soulever le paravent 1 ou de coincer le patient en cas d'élévation de la table d'examen sur laquelle ledit patient est allongé.

Les parties de base 15a et d'extension d'extrémité 15b de la tablette 15 peuvent par exemple être réalisées en acier inoxydable d'équivalence plomb de 0,20 mm minimum.

A titre de variante, la tablette 15 peut être prévue non télescopique et réalisée en matériau souple ou semi-rigide.

La partie supérieure 22 de la structure de paroi avant 2 est montée pivotante autour de l'axe pivot vertical 7, sur la bordure d'angle 4, au dessus de la bordure supérieure 214 de la partie inférieure 21 de la structure de paroi avant 2.

Cette partie supérieure pivotante 22 est délimitée par :
- une bordure latérale libre 221,
- une bordure latérale 222 formant une partie de la bordure d'angle 4,
- une bordure supérieure 223,
- une bordure inférieure 224,
- une face intérieure 225 orientée vers l'espace destiné au positionnement de l'opérateur, et
- une face extérieure opposée 226.
Et en outre cette partie supérieure pivotante 22 est constituée d'un panneau supérieur 227 associé à un panneau inférieur 228, ce panneau inférieur 228 étant prévu mobile en translation verticale par rapport audit panneau supérieur 227, pour réaliser une partie supérieure 22 de structure de paroi avant 2 télescopique, réglable en hauteur.

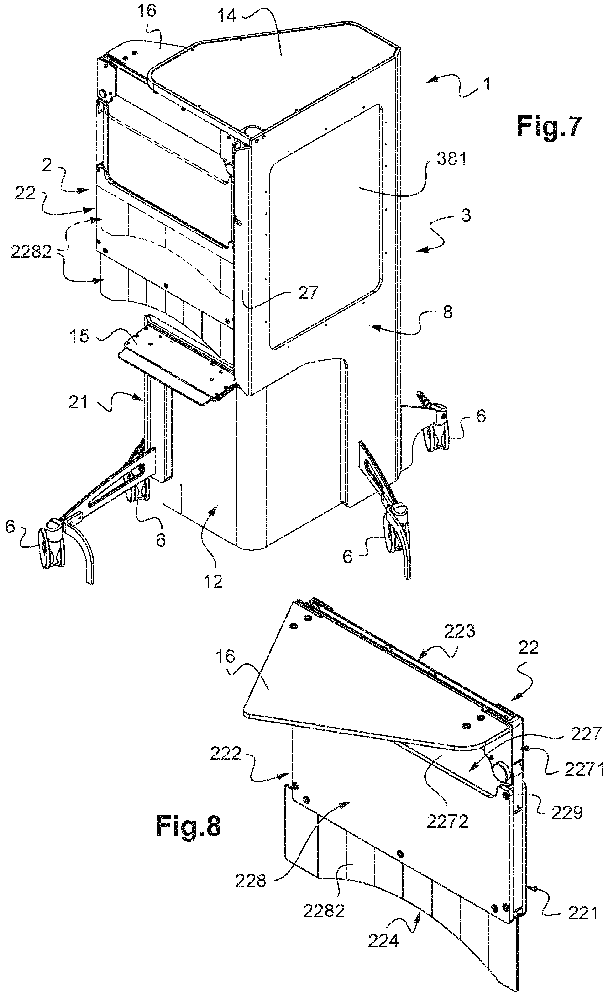

Comme on peut le voir sur les figures 8 à 10, le panneau supérieur 227 comporte une partie supérieure 2271 réalisée au moins partiellement en matériau métallique radio-protecteur tel que de l'acier d'équivalence plomb d'au minimum 0,20 mm, prolongée vers le bas par une partie inférieure 2272 en matériau radio-protecteur transparent, par exemple formée d'un panneau en plastique plombé type « Kyowaglass » (marque déposée), plastique radio-protecteur, type « Novashield glass » (marque déposée), verre au plomb, ou autre, le tout d'équivalence plomb entre 0,2 mm et 3 mm.

Des profilés métalliques de finition sont prévus sur les bordures inférieures de cette partie inférieure 2272, et sur les bordures de côté de cette partie inférieure 2272, entre la partie supérieure 2271 et ladite bordure inférieure.

Le panneau inférieur 228 comporte pour sa part une partie supérieure 2281, prolongée vers le bas par une partie inférieure 2282.

La partie supérieure 2281 se présente sous la forme de deux panneaux parallèles 2281a et 2281b réalisés en matériau radio-protecteur transparent tel que du plastique plombé type « Kyowaglass » (marque déposée), plastique radio-protecteur, type « Novashield glass » (marque déposée), verre au plomb, ou autre, le tout d'équivalence plomb entre 0,2 mm et 3 mm, qui sont espacés l'un de l'autre d'une distance correspondant, au jeu près, à l'épaisseur du panneau constituant la partie inférieure 2272 du panneau supérieur 227.

Les deux panneaux parallèles 2281a et 2281b sont assemblés par des profilés-entretoises qui équipent leurs deux bordures de côté verticales et leur bordure inférieure horizontale.

Ces deux panneaux parallèles 2281a et 2281b, constitutifs de la partie supérieure 2281, prennent en sandwich le panneau 2272 et ils ont la possibilité de se déplacer en translation verticale par rapport audit panneau 2272, pour obtenir le caractère télescopique et réglable en hauteur de la partie supérieure 22 de la structure de paroi avant 2.

Pour cela, le panneau inférieur 228 est suspendu au panneau supérieur 227 par l'intermédiaire d'un système d'équilibrage, ici en forme de deux ressorts spirale latéraux 229 du type à force constante.

Ces ressorts spirale 229 sont montés sur les côtés du panneau supérieur 227 et du panneau inférieur 228. Ils consistent en une bande métallique - dont une extrémité est enroulée en spirale et fixée sur le panneau supérieur 227 (autour d'un axe s'étendant perpendiculairement au plan dudit panneau 227), - et dont l'autre extrémité est fixée sur le chant de la bordure de côté en regard du panneau inférieur 228.

La force du système d'équilibrage 229 est adaptée pour permettre à un opérateur de réaliser manuellement les déplacements en montée ou en descente du panneau inférieur 228 par rapport au panneau supérieur 227. Du fait de la force constante de ces ressorts 229, une fois déplacé, le panneau 228 reste maintenu dans sa position.

Les figures 1, 2, 4, 7 (en pointillés) et 8 à 10 illustrent le panneau 228 en position haute maximale ; les figures 7, 11 et 12, illustrent le panneau 228 en position basse maximale.

La partie inférieure 2282 du panneau inférieur 228 est constituée par un rideau flexible formée d'une juxtaposition d'une pluralité de bandes souples en matériau radio-protecteur. La bordure supérieure du rideau flexible 2282 est fixée sur la bordure inférieure de la partie supérieure 2281 ; et sa bordure inférieure constitue la bordure inférieure 224 de la partie supérieure pivotante 22.

Ici, on remarque que cette bordure inférieure 224 est en forme d'arc de cercle pour suivre les contours généraux d'un patient allongé sur une table d'examen.

A titre de variante, cette bordure inférieure 224 peut être droite.

Ce rideau flexible 2282 peut s'étendre sur une hauteur de quelques centimètres ou de quelques dizaines de centimètres. Il est par exemple réalisé en matériau radio-protecteur tel que du PVC ou caoutchouc chargé de matériau radio-protecteur et d'une équivalence plomb de 0,125 mm au minimum.

Le rideau flexible 2282 peut être positionné pour venir en appui sur le corps d'un patient allongé sur une table d'examen afin d'optimiser la radioprotection ; il permet également le passage des bras de l'opérateur, dans le cas où il souhaite intervenir de l'autre côté de la partie supérieure pivotante 22.

La bordure supérieure 223 de la partie supérieure 22 de la structure de paroi avant 2 comporte un prolongement formant toiture 16, réalisé en matériau radio-protecteur, qui s'étend du côté de la face intérieure 225.

Ce prolongement 16 s'étend ici à l'équerre par rapport au plan de la structure de paroi avant 2 et sur toute la largeur de la bordure supérieure 223 ; il est avantageusement réalisé en matériau transparent, par exemple en plastique plombé type « Kyowaglass » (marque déposée), plastique radio-protecteur, type « Novashield glass » (marque déposée), verre au plomb, ou autre, le tout d'équivalence plomb comprise entre 0, 2 mm et 3 mm.

Le prolongement formant toiture 16 s'étend dans un plan décalé par rapport au plan du prolongement formant toiture 14 de la structure de paroi de côté 3 pour ne pas gêner le pivotement de la partie pivotante 22.

La forme et les dimensions des deux prolongements formant toiture 14 et 16 sont adaptées pour obtenir leur recouvrement partiel, et donc obtenir une continuité de radioprotection, qu'elle que soit la position admissible de la partie supérieure pivotante 22.

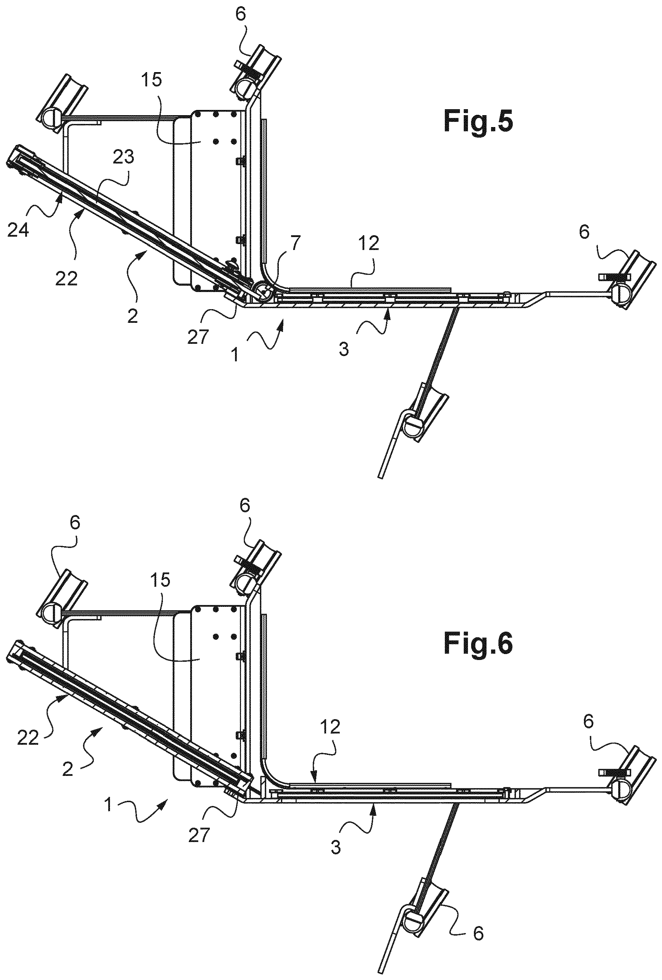

La partie supérieure 22 de la structure de paroi avant 2 est montée pivotante autour de l'axe pivot 7 sur la bordure d'angle 4 par l'intermédiaire d'un bras 23 visible sur les figures 5, 11 et 12.

Ce bras 23 consiste en un plat réalisé en acier, qui s'étend horizontalement à partir de la partie supérieure de la bordure d'angle 4.

L'une des extrémités du bras 23 est montée articulée autour de l'axe pivot vertical 7 sur la bordure latérale 32 de la structure de paroi de côté 3 ; et ce bras 23 vient s'insérer dans un logement de réception 24 ménagé dans la partie supérieure 2271 du panneau supérieur 227 de la partie pivotante 22, débouchant latéralement à cet effet, pour servir de support à l'ensemble formé par le panneau supérieur 227, le panneau inférieur 228 et le prolongement formant toiture 16.

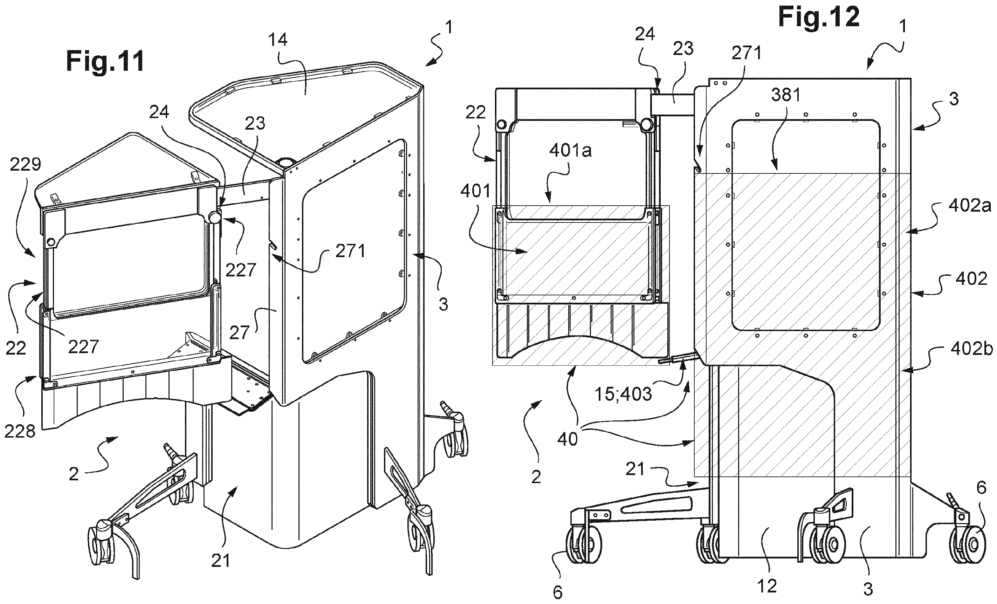

Cet ensemble formé par le panneau supérieur 227, le panneau inférieur 228 et le prolongement formant toiture 16 est monté mobile en translation horizontale sur le bras support 23 pour occuper :
- une position dite active, dans laquelle la bordure latérale 222 de la partie supérieure pivotante 22 est située au plus près de la bordure latérale en regard 32 de la structure de paroi de côté 3 (comme illustré sur les figures 1 à 7), et
- une position dite inactive dans laquelle la bordure latérale 222 de la partie supérieure pivotante 22 est située à distance de la bordure latérale en regard 32 de la structure de paroi de côté 3 (comme illustré sur les figures 11 et 12), permettant, comme expliqué ci-après, la pose d'un équipement de housse stérile.

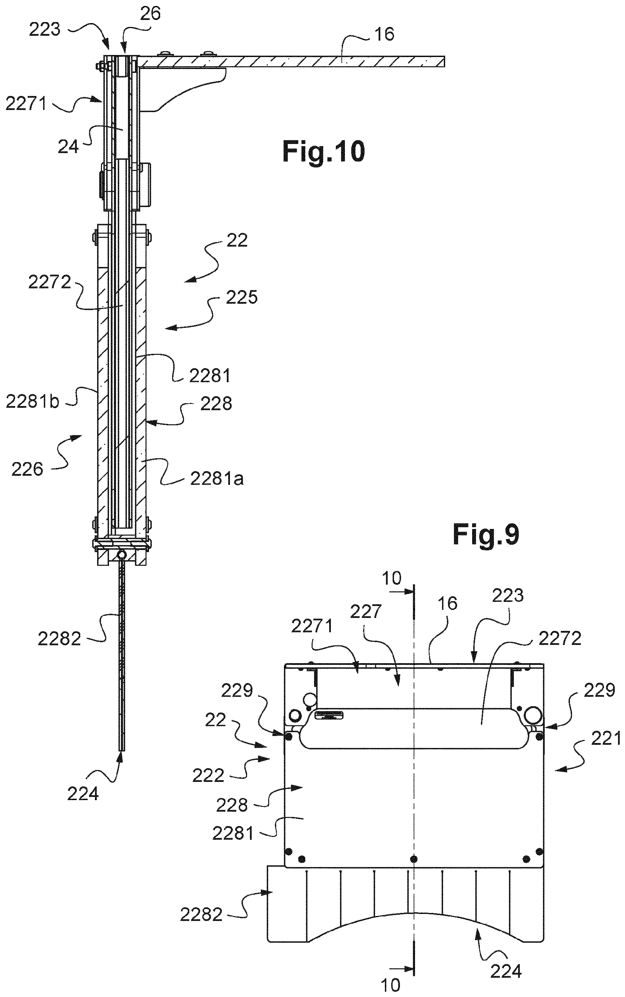

La partie supérieure du logement 24 est avantageusement équipée de galets 26 (visibles sur la figure 10), agencés pour rouler sur la bordure supérieure du bras support 23, et faciliter les manœuvres de coulissement correspondantes pour occuper les positions active et inactive précitées.

Des moyens peuvent également être prévus pour verrouiller de manière amovible ces positions active et inactive précitées. Ces moyens, non visibles sur les figures, peuvent consister en un système d'indexage, par exemple du type à bille(s) montée(s) sur ressort, portée(s) par l'un des éléments, coopérant avec un logement adapté ménagé sur l'autre élément.

Sur les figures 1 à 7 on remarque que la bordure latérale 32 de la partie supérieure 38 de la structure de paroi de côté 3, qui forme une partie de la bordure d'angle 4, comporte une extension en forme d'aile latérale 27 qui s'étend sur quelques centimètres de large. Cette aile latérale 27 est adaptée pour venir recouvrir la bordure latérale 222 en regard de la partie supérieure 22 de la structure de paroi avant 2, et l'axe pivot 7 associé, lorsque cette partie supérieure 22 est dans sa position active, et cela du côté de la face extérieure 226 de ladite partie supérieure 22, de manière à optimiser la radioprotection du paravent 1.

Uniquement à titre indicatif, la hauteur du paravent 1, c'est-à-dire la distance entre le plan d'appui au sol des roues 6 et les prolongements formant toiture 14, 16, peut être de l'ordre de 1,90 à 2,30 m. La largeur de la structure de paroi de côté 3 et celle des deux parties 21 et 22 de la structure de paroi avant 2 peut être inférieure ou égale à 0,80 m, par exemple de l'ordre de 0,60 à 0,80 m; et la bordure supérieure 214 de la partie inférieure 21 de la structure de paroi avant 2 peut s'étendre à une hauteur de l'ordre de 0,70 à 1,10 m.

La partie supérieure 22 de la structure de paroi avant 2 est montée pivotante autour de l'axe pivot 7 entre :
- une position dite fermée, illustrée sur la figure 7, dans laquelle cette partie supérieure 22 s'étend dans un plan parallèle ou sensiblement parallèle au plan dans lequel s'étend la partie inférieure 21, dans le prolongement ou sensiblement dans le prolongement de cette dernière, et
- une position d'ouverture maximale, illustrée sur les figures 1 à 6, dans laquelle cette partie supérieure 22 s'étend dans un plan décalé du côté de la face extérieure 216 de la partie inférieure 21.

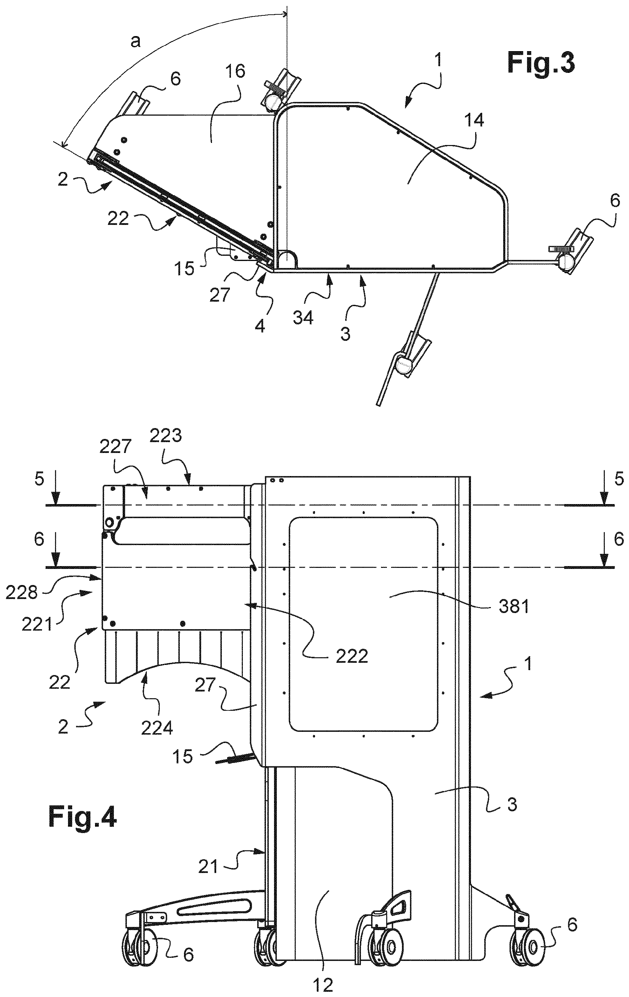

Le secteur angulaire de pivotement correspondant a (figure 3) peut être compris entre 50 et 90°, par exemple de l'ordre de 60°.

Dans le cadre d'une utilisation du paravent radio-protecteur 1 en salle d'opération, certaines de ses parties constitutives sont recouvertes par un équipement de housse stérile 40 tel qu'illustré sur la figure 12.

Cet équipement de housse stérile 40 est adapté pour recouvrir au moins une partie de la hauteur des faces intérieure et extérieure du paravent radio-protecteur 1 et il comprend ici:
- une partie de housse 401 adaptée pour venir recouvrir au moins partiellement la partie supérieure 22 de la structure de paroi avant 2,
- une partie de housse 402 adaptée pour venir recouvrir au moins partiellement la structure de paroi de côté 3 et la partie inférieure 21 de la structure de paroi avant 2, et
- une partie de housse 403 en forme de structure souple adaptée pour venir recouvrir au moins partiellement le prolongement de protection en forme de tablette 15, solidaire ou non de la partie de housse 402 précitée.

La partie de housse 401 est adaptée pour venir recouvrir le panneau inférieur 228 de la partie supérieure pivotante 22. Elle se présente sous la forme d'une enveloppe ou poche souple réalisée en matériau transparent, par exemple un film transparent en polypropylène, de forme générale carrée ou rectangulaire, et dont une bordure est munie d'une ouverture de positionnement 401a.

Après déplacement de la partie supérieure 22 en position inactive, telle qu'illustrée sur les figures 11 et 12 (c'est-à-dire avec sa bordure latérale 222 séparée de bordure latérale 32 en regard de la structure de paroi de côté 3), la poche souple 401 est positionnée par engagement de la bordure inférieure 224 de la partie supérieure 22 dans l'ouverture 401a ; et une fois la poche souple 401 correctement positionnée autour du panneau inférieur 228, des moyens de fixation adaptés, du type bandes adhésives, ventouses, élastiques ou autres, assurent son maintien en position.

Un tel positionnement est rendu possible par la mise en position inactive de la partie supérieure 22 du paravent radio-protecteur 1. Bien entendu l'utilisation ultérieure du paravent nécessite sa remise préalable en position active.

La partie de housse 402 consiste en au moins un panneau de matière souple adapté pour venir recouvrir une partie de la hauteur des parties inférieures 21 et 37 des structures de paroi avant 2 et de côté 3, et aussi une partie de la hauteur de la partie supérieure 38 de la paroi de côté 3.

En l'occurrence la partie de housse 402 consiste ici en un panneau unique constitué de l'assemblage de plusieurs panneaux de matière différente, a savoir un panneau 402a en matière transparente, par exemple en polypropylène destiné à venir se positionner en regard de la partie transparente 381 de ladite structure de paroi de côté 3, et un panneau 402b en matériau non tissé opaque, par exemple en polypropylène, destiné a venir se positionner sur lesdites parties inférieures 21 et 37 des structures de paroi avant 2 et de côté 3.

Le positionnement de cette partie de housse 402 s'effectue par simple enveloppement des zones concernées du paravent radio-protecteur 1. Pour faciliter l'initiation de cette opération, le panneau de matière 402 peut comporter une structure d'accrochage sur une bordure d'extrémité, adaptée pour venir s'accrocher sur une structure de crochetage 271 prévue sur le châssis 8 du paravent 1, par exemple sur la hauteur de l'aile de recouvrement 27.

Une fois cette partie de housse 402 correctement positionnée, des moyens de fixation adaptés, du type bandes adhésives, ventouses ou autres, assurent son maintien en position.

La partie de housse 403 adaptée pour venir recouvrir le prolongement de protection en forme de tablette 15 peut se présenter sous la forme d'une extension solidaire de la partie de housse précitée 402 munie de moyens de fixation adaptés, du type bandes adhésives, ventouses, élastiques ou autres assurant son maintien en position.

A titre de variante, cette partie de housse 403 peut se présenter sous la forme d'une enveloppe ou poche indépendante réalisée en matériau souple, par exemple en polypropylène, de forme générale rectangulaire et dont une bordure est munie d'une ouverture de positionnement.

Cette poche souple 403 est alors positionnée par engagement de l'extrémité du prolongement de protection en forme de tablette 15 dans son ouverture ; et une fois correctement positionnée, des moyens de fixation adaptés, du type bandes adhésives, ventouses ou autres, assurent son maintien en position.

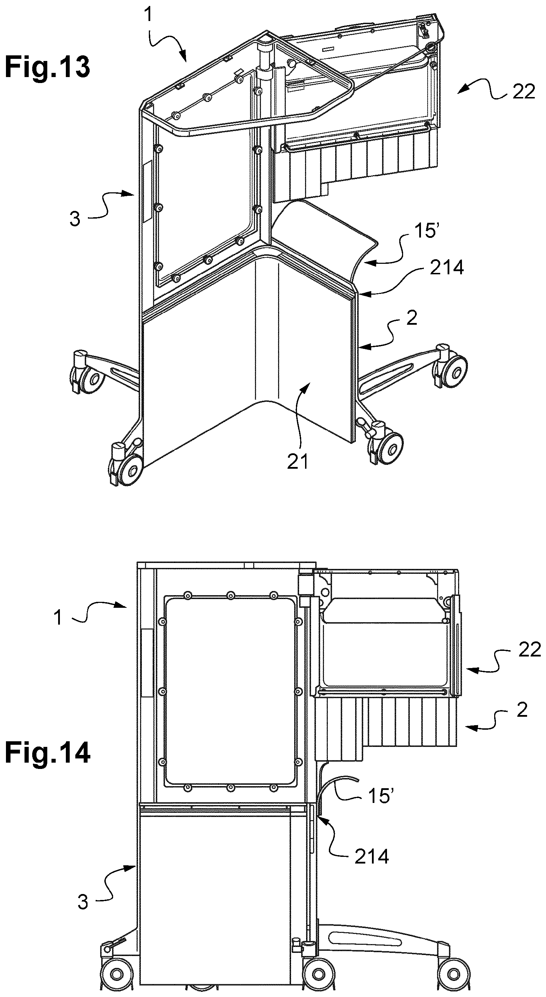

Encore à titre de variante, la tablette 15 illustrée sur le mode de réalisation des figures 1 à 12 peut être remplacée par une bavette semi-rigide, tel que représenté sur les figures 13 et 14.

Comme on peut le voir sur ces figures 13 et 14, la bavette semi-rigide 15' s'étend vers le haut, à partir de la bordure supérieure 214 de la partie inférieure 21 de la structure de paroi avant 2, et en porte à faux du côté de la face extérieure 216.

Elle consiste en un panneau monobloc en matériau radio-protecteur (par exemple du PVC ou du caoutchouc chargé de plomb ou autre matériau radio-protecteur), qui est fixé le long de ladite bordure supérieure 214 et dont la bordure en porte à faux est libre. Cette bavette 15' est à section courbe ou en arc de cercle au repos.

Du fait de sa constitution semi-rigide, sa forme et son positionnement peuvent être modifiés, volontairement par l'opérateur, ou du fait d'un contact avec un appareil ou élément de l'environnement (patient, table support du patient...).

Le paravent radio-protecteur 1 selon l'invention est particulièrement adapté pour protéger un ou plusieurs opérateurs dans une salle d'opération intégrant un ou plusieurs équipements émetteur(s) de rayonnements ionisants, notamment un appareil de fluoroscopie.

Ce paravent radio-protecteur 1 peut être facilement déplacé par simple roulement sur le sol pour positionner correctement ses structures de parois avant 2 et de côté 3 entre l'opérateur et l'équipement émetteur de rayonnements ionisants. Comme illustré sur la figure 2, il peut être positionné au plus près d'une table d'examen T sur laquelle est allongé un patient, avec sa structure de paroi avant 2 disposée le long de cette table T.

Alors, l'opérateur peut déplacer manuellement la partie supérieure pivotante 22 et la partie mobile en translation verticale 228, pour assurer son confort gestuel et optimiser la radioprotection, en fonction de l'intervention qu'il doit réaliser sur le patient.

En particulier, on comprend que le pivotement vers l'avant de la partie supérieure pivotante 22 permet d'assurer une radioprotection efficace, avec le rideau souple 2282 en appui contre le patient, tout en libérant une large ouverture au dessus du patient, devant la partie inférieure 21 de la structure de paroi avant 2, facilitant l'intervention de l'opérateur.

Ce paravent mobile sur roues n'a aucune interaction avec le praticien ; il permet aussi à l'opérateur d'intervenir sur le patient sans entrave en cas d'urgence.

D'autre part, on comprend également que le contact de certaines parties mobiles d'équipements (par exemple l'arceau de l'équipement de fluoroscopie), avec le panneau souple 12 ou encore avec la partie supérieure pivotante 22, n'entraine pas de perturbations majeures du positionnement général du paravent radio-protecteur 1.

## Revendications

1. Paravent de radioprotection (1) pour la protection d'au moins un opérateur à l'encontre des rayonnements ionisants, lequel paravent (1) comprend une structure de paroi avant (2) en matériau(x) radio-protecteur(s) et une structure de paroi de côté (3) en matériau(x) radio-protecteur(s), reliées entre elles au niveau d'une bordure d'angle (4) verticale ou sensiblement verticale, lequel paravent (1) comporte un piètement (5) muni de roues (6) d'appui au sol,
ladite structure de paroi avant (2) comprenant une partie inférieure (21) et une partie supérieure (22) mobiles l'une par rapport à l'autre, laquelle partie supérieure (22) de ladite structure de paroi avant (2) est montée pivotante au niveau de ladite bordure d'angle (4), autour d'un axe pivot (7) vertical ou sensiblement vertical,
laquelle structure de paroi de côté (3) s'étend dans un plan vertical ou sensiblement vertical et comporte : - une bordure latérale libre (31), - une bordure latérale (32) définissant une partie de ladite bordure d'angle (4), - une bordure inférieure (33), et - une bordure supérieure (34),
laquelle structure de paroi de côté (3) comporte une partie supérieure (38) dont au moins une partie (381) est réalisée en matériau radio-protecteur transparent, et une partie inférieure (37),
la partie inférieure (21) de la structure de paroi avant (2) étant délimitée par : - une bordure latérale libre (211), - une bordure latérale (212) définissant une partie de ladite bordure d'angle (4), - une bordure inférieure (213), et - une bordure supérieure (214), laquelle partie inférieure (21) de la structure de paroi avant (2) s'étend dans un plan vertical ou sensiblement vertical qui est décalé par rapport au plan de ladite structure de paroi de côté (3), d'un angle fixe compris entre 70 et 120°, de préférence de l'ordre 90°, **caractérisé en ce qu'**il comporte une partie (12) réalisée en matériau radio-protecteur souple, qui se présente sous la forme d'un panneau s'étendant :
- sur une partie de la hauteur de ladite bordure d'angle (4), et
- sur une partie de ladite partie inférieure (37) de la structure de paroi de côté (3), ainsi que sur une partie de ladite partie inférieure (21) de la structure de paroi avant (2), de part et d'autre de ladite bordure d'angle (4).

2. Paravent selon la revendication 1, **caractérisé en ce que** ledit panneau (12) en matériau radio-protecteur souple s'étend :
- à partir de la bordure inférieure (33) de ladite partie inférieure (37) de la structure de paroi de côté (3) et de la bordure inférieure (213) ladite partie inférieure (21) de la structure de paroi avant (2), sur plus de la moitié de la hauteur desdites parties inférieures (33, 21) de la structure de paroi de côté (3) et de la structure de paroi avant (2), et
- à partir de ladite bordure d'angle (4), sur plus de la moitié de la largeur desdites parties inférieures (21, 37) des structures de parois avant (2) et de côté (3).

3. Paravent selon l'une quelconque des revendications 1 ou 2, **caractérisé en ce que** la structure de paroi de côté (3) comporte une face intérieure (35) orientée vers l'espace de positionnement de l'opérateur, et une face extérieure (36) opposée,
et **en ce que** la bordure supérieure (34) de ladite structure de paroi de côté (3) comporte un prolongement (14) formant toiture, en matériau radio-protecteur, qui s'étend du côté de ladite face intérieure (35).

4. Paravent selon l'une quelconque des revendications 1 à 3, **caractérisé en ce que** la partie inférieure (21) de la structure de paroi avant (2) comporte :
- une face intérieure (215) orientée vers l'espace de positionnement de l'opérateur,
- une face extérieure (216) opposée, et
- une bordure supérieure (214),
et **en ce que** ladite bordure supérieure (214) comporte un prolongement de protection (15, 15'), en forme de tablette (15) ou de bavette semi-rigide (15'), réalisé en matériau radio-protecteur, qui s'étend du côté de ladite face extérieure (216).

5. Paravent selon la revendication 4, **caractérisé en ce que** ladite tablette (15) s'étend dans une position horizontale ou sensiblement horizontale à partir de la bordure supérieure (214) de la partie inférieure (21) de la structure de paroi avant (2),
**en ce que** ladite tablette (15) est montée pivotante sur ladite bordure supérieure (214) de la partie inférieure (21) de la structure de paroi avant (2), pour permettre son relevage à partir de ladite position horizontale,
et **en ce que** ladite tablette (15) comporte une extension d'extrémité (15a) rétractable pour la rendre télescopique.

6. Paravent selon la revendication 1, **caractérisé en ce que** la partie supérieure (22) de la structure de paroi avant (2) comporte : - une bordure latérale libre (221), - une bordure latérale (222) formant une partie de ladite bordure d'angle (4), - une bordure supérieure (223), - une bordure inférieure (224), - une face intérieure (225) orientée vers l'espace de positionnement de l'opérateur, et - une face extérieure (226) opposée,
laquelle partie supérieure (22) de la structure de paroi avant (2) est constituée d'au moins un panneau en matériau radio-protecteur.

7. Paravent selon la revendication 6, **caractérisée en ce que** la bordure latérale (32) de la structure de paroi de côté (3) qui forme une partie de ladite bordure d'angle (4) comporte une extension en forme d'aile latérale (27) adaptée pour venir recouvrir la bordure latérale (222) en regard de la partie supérieure (22) de la structure de paroi avant (2), et l'axe pivot associé (7), ce recouvrement étant réalisé du côté de la face extérieure (226) de ladite partie supérieure (22) de la structure de paroi avant (2).

8. Paravent selon la revendication 3 prise en combinaison avec l'une quelconque des revendications 6 ou 7, **caractérisé en ce que** la bordure supérieure (223) de la partie supérieure (22) de la structure de paroi avant (2) comporte un prolongement formant toiture (16), réalisé en matériau radio-protecteur, qui s'étend du côté de la face intérieure (225) de ladite partie supérieure (22) de la structure de paroi avant (2), dans un plan décalé par rapport au plan du prolongement formant toiture (14) de la structure de paroi de côté (3).

9. Paravent selon l'une quelconque des revendications 6 à 8, **caractérisé en ce que** la bordure inférieure (224) de la partie supérieure (22) de la structure de paroi avant (2) est constituée par un rideau flexible (2282) formé d'une juxtaposition d'une pluralité de bandes souples en matériau radio-protecteur.

10. Paravent selon l'une quelconque des revendications 6 à 9, **caractérisé en ce que** la partie supérieure (22) de la structure de paroi avant (2) comprend :
- un panneau supérieur (227) dont au moins une partie est réalisée en matériau radio-protecteur transparent, et
- un panneau inférieur (228), dont au moins une partie est réalisée en matériau radio-protecteur transparent,
lequel panneau inférieur (228) est mobile en translation verticale par rapport audit panneau supérieur (227), pour réaliser une partie supérieure (22) de structure de paroi avant (2) télescopique, réglable en hauteur.

11. Paravent selon la revendication 10 **caractérisé en ce que** ledit panneau inférieur (228) mobile est solidaire dudit panneau supérieur (227) par l'intermédiaire d'un système d'équilibrage (229), par exemple du type ressort spirale à force constante (229).

12. Paravent selon l'une quelconque des revendications 6 à 11, **caractérisée en ce que** la partie supérieure (22) de la structure de paroi avant (2) comprend un bras support (23) monté pivotant sur ladite bordure d'angle (4) autour dudit axe pivot (7),
et **en ce que** ledit au moins un panneau (227, 228)est monté mobile en translation horizontale sur ledit bras support (23).

13. Équipement en forme de housse (40) destiné à recouvrir au moins une partie de la hauteur d'un paravent radio-protecteur (1) selon la revendication 12, **caractérisé en ce qu'**il comprend :
- une poche souple (401) munie d'une ouverture (401a), laquelle poche souple (401) est adaptée pour venir recouvrir au moins partiellement la partie supérieure (22) de la structure de paroi avant (2) par engagement de la bordure inférieure(224) de cette dernière dans ladite ouverture (401a) de ladite poche souple (401), laquelle poche souple (401) est munie d'au moins une partie transparente et de moyens pour sa fixation sur ladite partie supérieure (22) de la structure de paroi avant (2), et
- au moins un panneau souple (402) adapté pour venir recouvrir au moins partiellement ladite structure de paroi de côté (3) et la partie inférieure (21) de ladite structure de paroi avant (2),
ledit au moins un panneau souple (402) comportant au moins une partie transparente destinée à venir se positionner en regard de la partie transparente (381) de ladite structure de paroi de côté (3), et des moyens de fixation sur lesdites structures de paroi de côté (3) et de paroi avant (2), et
- en cas de présence d'un tablette (15) prolongeant la bordure supérieure (214) de la partie inférieure (21) de la structure de paroi avant, une structure souple (403) de recouvrement au moins partiel de ladite tablette (15), soit solidaire dudit panneau (402) pour le recouvrement de ladite structure de paroi de côté (3) et de la partie inférieure (21) de la structure de paroi avant (2), soit indépendante dudit panneau (402), laquelle structure souple (403) est munie de moyens pour sa fixation sur ladite tablette (15).

## Patentansprüche

1. Strahlenschutzschirm (1) für den Schutz wenigstens eines Bedieners vor ionisierenden Strahlen, wobei der Schutzschirm (1) eine Vorderwandstruktur (2) aus Strahlenschutzmaterial und eine Seitenwandstruktur (3) aus Strahlenschutzmaterial aufweist, die im Bereich eines senkrechten oder im Wesentlichen senkrechten Eckrands (4) miteinander verbunden sind, wobei der Schutzschirm (1) ein mit Rädern (6) zum Abstützen am Boden versehenes Grundgestell (5) aufweist,
wobei die Vorderwandstruktur (2) einen unteren Teil (21) und einen oberen Teil (22), die zueinander beweglich sind, aufweist, wobei der obere Teil (22) der Vorderwandstruktur (2) im Bereich des Eckrands (4) um eine senkrechte oder im Wesentlichen senkrechte Schwenkachse (7) schwenkbar montiert ist,
wobei sich die Seitenwandstruktur (3) in einer senkrechten oder im Wesentlichen senkrechten Ebene erstreckt und - einen freien Seitenrand (31), - einen einen Teil des Eckrands (4) definierenden Seitenrand (32), - einen unteren Rand (33) und - einen oberen Rand (34) aufweist,
wobei die Seitenwandstruktur (3) einen oberen Teil (38), von dem mindestens ein Teil (381) aus einem durchsichtigen Strahlenschutzmaterial gefertigt ist, und einen unteren Teil (37) aufweist,
wobei der untere Teil (21) der Vorderwandstruktur (2) durch : - einen freien Seitenrand (211), - einen einen Teil des Eckrands (4) definierenden Seitenrand (212), - einen unteren Rand (213) und - einen oberen Rand (214) abgegrenzt ist, wobei sich der untere Teil (21) der Vorderwandstruktur (2) in einer senkrechten oder im Wesentlichen senkrechten Ebene erstreckt, die gegenüber der Ebene der Seitenwandstruktur (3) um einen festen Winkel zwischen 70° und 120°, vorzugsweise um etwa 90°, versetzt ist,
**dadurch gekennzeichnet, daß** er einen aus einem weichen Strahlenschutzmaterial gefertigten Teil (12) aufweist, der die Form einer Platte hat, die sich
- über einen Teil der Höhe des Eckrands (4) und
- über einen Teil des unteren Teils (37) der Seitenwandstruktur (3), sowie über einen Teil des unteren Teils (21) der Vorderwandstruktur (2) beiderseits des Eckrands (4)
erstreckt.

2. Schutzschirm gemäß Anspruch 1, **dadurch gekennzeichnet, daß** sich die Platte (12) aus weichem Strahlenschutzmaterial
- vom unteren Rand (33) des unteren Teils (37) der Seitenwandstruktur (3) und vom unteren Rand (213) des unteren Teils (21) der Vorderwandstruktur (2) aus über mehr als die Hälfte der Höhe der unteren Teile (33, 21) der Seitenwandstruktur (3) und der Vorderwandstruktur (2) und
- vom Eckrand (4) aus über mehr als die Hälfte der Breite der unteren Teile (21, 37) der Vorder- (2) und der Seitenwandstruktur (3)
erstreckt.

3. Schutzschirm gemäß einem der Ansprüche 1 oder 2, **dadurch gekennzeichnet, daß** die Seitenwandstruktur (3) eine zum Aufstellraum des Bedieners hin gerichtete Innenseite (35) und eine abgewandte Außenseite (36) aufweist und daß der obere Rand (34) der Seitenwandstruktur (3) eine ein Dach bildende Verlängerung (14) aus Strahlenschutzmaterial aufweist, die sich auf der Seite der Innenseite (35) erstreckt.

4. Schutzschirm gemäß einem der Ansprüche 1 bis 3, **dadurch gekennzeichnet, daß** der untere Teil (21) der Vorderwandstruktur (2)
- eine zum Aufstellraum des Bedieners hin gerichtete Innenseite (215),
- eine abgewandte Außenseite (216) und
- einen oberen Rand (214)
aufweist
und daß der obere Rand (214) eine Schutzverlängerung (15, 15') in Form eines Bretts (15) oder einer halbsteifen Ablage (15') aus Strahlenschutzmaterial aufweist, die sich auf der Seite der Außenseite (216) erstreckt.

5. Schutzschirm gemäß Anspruch 4, **dadurch gekennzeichnet, daß** sich das Brett (15) in horizontaler oder im Wesentlichen horizontaler Stellung vom oberen Rand (214) des unteren Teils (21) der Vorderwandstruktur (2) aus erstreckt,
daß das Brett (15) am oberen Rand (214) des unteren Teils (21) der Vorderwandstruktur (2) schwenkbar angebracht ist, um dessen Hochklappen aus der horizontalen Stellung zu ermöglichen,
und daß das Brett (15) ein ausziehbares Ende (15a) aufweist, um es ausziehbar zu machen.

6. Schutzschirm gemäß Anspruch 1, **dadurch gekennzeichnet, daß** der obere Teil (22) der Vorderwandstruktur (2) - einen freien seitlichen Rand (221), - einen seitlichen Rand (222), der einen Teil des Eckrands (4) bildet, - einen oberen Rand (223), - einen unteren Rand (224), - eine zum Aufstellraum des Bedieners hin gerichtete Innenseite (225) und - eine abgewandte Außenseite (226) aufweist,
wobei der obere Teil (22) der Vorderwandstruktur (2) aus wenigstens einer Platte aus Strahlenschutzmaterial besteht.

7. Schutzschirm gemäß Anspruch 6, **dadurch gekennzeichnet, daß** der seitliche Rand (32) der Seitenwandstruktur (3), der einen Teil des Eckrands (4) bildet, eine Verlängerung in Form eines Seitenflügels (27) aufweist, der dazu ausgelegt ist, den seitlichen Rand (222) zum oberen Teil (22) der Vorderwandstruktur (2) hin und die zugehörige Schwenkachse (7) zu bedecken, wobei diese Bedeckung auf der Seite der Außenseite des oberen Teils (22) der Vorderwandstruktur (2) ausgeführt ist.

8. Schutzschirm gemäß Anspruch 3 zusammen mit einem der Ansprüche 6 oder 7, **dadurch gekennzeichnet, daß** der obere Rand (223) des oberen Teils (22) der Vorderwandstruktur (2) eine ein aus Strahlenschutzmaterial gefertigtes Dach (16) bildende Verlängerung aufweist, die sich auf der Seite der Innenseite (225) des oberen Teils (22) der Vorderwandstruktur (2) in einer gegenüber der Ebene der ein Dach (14) der Seitenwandstruktur (3) bildenden Verlängerung versetzten Ebene erstreckt.

9. Schutzschirm gemäß einem der Ansprüche 6 bis 8, **dadurch gekennzeichnet, daß** der untere Rand (224) des oberen Teils (22) der Vorderwandstruktur (2) aus einer flexiblen Gardine (2282) besteht, die durch ein Aneinanderreihen von weichen Bändern aus Strahlenschutzmaterial gebildet ist.

10. Schutzschirm gemäß einem der Ansprüche 6 bis 9, **dadurch gekennzeichnet, daß** der obere Teil (22) der Vorderwandstruktur (2)
- eine obere Platte (227), von der wenigstens ein Teil aus durchsichtigem Strahlenschutzmaterial gefertigt ist, und
- eine untere Platte (228), von der wenigstens ein Teil aus durchsichtigem Strahlenschutzmaterial gefertigt ist,
aufweist,
wobei die untere Platte (228) gegenüber der oberen Platte (227) senkrecht verschiebbar ist, um einen ausziehbaren, in der Höhe verstellbaren oberen Teil (22) der Vorderwandstruktur (2) zu bilden.

11. Schutzschirm gemäß Anspruch 10, **dadurch gekennzeichnet, daß** die bewegliche untere Platte (228) mit der oberen Platte (227) durch ein Ausgleichssystem (229), zum Beispiel vom Typ einer Spiralfeder mit konstanter Kraft (229), verbunden ist.

12. Schutzschirm gemäß einem der Ansprüche 6 bis 11, **dadurch gekennzeichnet, daß** der obere Teil (22) der Vorderwandstruktur (2) einen Stützarm (23) aufweist, der am Eckrand (4) um die Schwenkachse (7) schwenkend angebracht ist,
und daß die wenigstens eine Platte (227, 228) auf dem Stützarm (23) horizontal verschiebbar angebracht ist.

13. Ausrüstung in Form einer Hülle (40), die dazu bestimmt ist, wenigstens einen Teil der Höhe eines Strahlenschutzschirms gemäß Anspruch 12 zu bedecken, **dadurch gekennzeichnet, daß** sie
- eine weiche, mit einer Öffnung (401a) versehene Tasche (401), die (401) dazu ausgelegt ist, den oberen Teil (22) der Vorderwandstruktur (2) wenigstens teilweise durch Einbringen des unteren Rands (224) der letzteren in die Öffnung (401a) der weichen Tasche (401) zu bedecken, wobei die weiche Tasche (401) mit wenigstens einem durchsichtigen Teil und mit Mitteln für deren Befestigung am oberen Teil (22) der Vorderwandstruktur (2) versehen ist, und
- wenigstens eine weiche Platte (402), die dazu ausgelegt ist, die Seitenwandstruktur (3) und den unteren Teil (21) der Vorderwandstruktur (2) wenigstens teilweise zu bedecken,
wobei die wenigstens eine weiche Platte (402) wenigstens einen durchsichtigen Teil, der vor den durchsichtigen Teil (381) der Seitenwandstruktur (3) kommen soll, und Mittel zum Befestigen an der Seitenwand- (3) und der Vorderwandstruktur (2) aufweist, und
- falls es ein den oberen Rand (214) des unteren Teils (21) der Vorderwandstruktur verlängernden Bretts (15) gibt, eine weiche Struktur (403) zum wenigstens teilweisen Abdecken des Bretts (15), die entweder an der Platte (402) zum Abdecken der Seitenwandstruktur (3) und des unteren Teils (21) der Vorderwandstruktur (2) fest ist oder von der besagten Platte (402) unabhängig ist, wobei die weiche Struktur (403) mit Mitteln für deren Befestigung am Brett (15) versehen ist,
aufweist.

## Claims

1. Radioprotective screen (1) for protecting at least one operator from ionizing radiation, said screen (1) comprising a front wall structure (2) made of radioprotective material(s) and a lateral wall structure (3) made of radioprotective material(s) linked to one another at a vertical or essentially vertical corner edge (4), said screen (1) comprising a base (5) provided with ground support wheels (6),
said front wall structure (2) comprising a lower part (21) and an upper part (22) which can be moved relative to one another, said upper part (22) of said front wall structure (2) being mounted pivotally around a vertical or essentially vertical pivoting axis (7) at said corner edge (4), the lateral wall structure (3) extending in a vertical or essentially vertical plane and comprising: - a free lateral edge (31), - a lateral edge (32) defining a part of said corner edge (4), - a lower edge (33), and - an upper edge (34),
said lateral wall structure (3) comprising an upper part (38), at least a part (381) of which is made of transparent radioprotective material, and a lower part (37),
the lower part (21) of the front wall structure (2) being defined by: - a free lateral edge (211), - a lateral edge (212) defining a part of said corner edge (4), - a lower edge (213), and - an upper edge (214),
said lower part (21) of the front wall structure (2) extending in a vertical or essentially vertical plane which is offset with respect to the plane of said lateral wall structure (3) by a fixed angle between 70° and 120°, preferably in the order of 90°,
**characterized in that** it comprises a part (12) made of flexible radioprotective material which is shaped as a panel that extends:
- over a part of the height of said corner edge (4), and
- over a part of said lower part (37) of the lateral wall structure (3) as well as over a part of the lower part (21) of the front wall structure (2) at both sides of said corner edge (4).

2. Screen according to claim 1, **characterized in that** said panel (12) made of flexible radioprotective material extends:
- from the lower edge (33) of said lower part (37) of the lateral wall structure (3), and from the lower edge (213) of the lower part (21) of the front wall structure (2), over more than half of the height of said lower parts (33, 21) of the lateral wall structure (3) and of the front wall structure (2), and
- from said corner edge (4), over more than half of the width of said lower parts (21, 37) of the front (2) and lateral (3) wall structure.

3. Screen according to anyone of claims 1 or 2, **characterized in that** the lateral wall structure (3) comprises an inner face (35) turned towards the positioning space of the operator, and an opposite outer face (36), and **in that** the upper edge (34) of the lateral wall structure (3) comprises an extension (14) forming a roof made of radioprotective material, which extends on the side of said inner face (35).

4. Screen according to anyone of claims 1 to 3, **characterized in that** the lower part (21) of the front wall structure (2) comprises:
- an inner face (215) turned towards the positioning space of the operator,
- an opposite outer face (216), and
- an upper edge (214),
and **in that** said upper edge (214) comprises a protective extension (15, 15') shaped as a tablet (15) or a semi-rigid bib (15'), made of a radioprotective material, which extends on the side of said outer face (216).

5. Screen according to claim 4, **characterized in that** said tablet (15) extends in a horizontal or essentially horizontal position from the upper edge (214) of the lower part (21) of the front wall structure (2),
**in that** said tablet (15) is mounted pivotally on said upper edge (214) of the lower part (21) of the front wall structure (2), in order to allow it to be raised from said horizontal position,
and **in that** said tablet (15) comprises a retractable end extension (15a) for making it telescopic.

6. Screen according to claim 1, **characterized in that** the upper part (22) of the front wall structure (2) comprises: - a free lateral edge (221), - a lateral edge (222) defining a part of said corner edge (4), - an upper edge (223), a lower edge (224), an inner face (225) turned towards the positioning space of the operator, and - an opposite outer face (226).

7. Screen according to claim 6, **characterized in that** lateral edge (32) of the lateral wall structure (3), which forms a part of said corner edge (4), comprises an extension shaped as a lateral wing (27) able to cover the lateral edge (222) which is in front of the upper part (22) of the front wall structure (2), and the associated pivoting axis (7), the cover being formed on the side of the outer face (226) of the upper part (22) of the front wall structure (2).

8. Screen according to claim 3 together with anyone of claims 6 or 7, **characterized in that** the upper edge (223) of the upper part (22) of the front wall structure (2) comprises a roof-forming extension (16) made of radioprotective material, which extends on the side of the inner face (225) of said upper part (22) of the front wall structure (2) in a plane that is offset with respect to the plane of the roof-forming extension (14) of the lateral wall structure (3).

9. Screen according to anyone of claims 6 to 8, **characterized in that** the lower edge (224) of the upper part (22) of the front wall structure (2) is formed by a flexible curtain (2282) composed by a juxtaposition of a plurality of flexible bands made of radioprotective material.

10. Screen according to anyone of claims 6 to 9, **characterized in that** the upper part (22) of the front wall structure (2) comprises:
- an upper panel (227) at least a part of which is made of radioprotective material, and
- a lower panel (228) at least part of which is made of a transparent radioprotective material,
said lower panel (228) being vertically translationally movable with respect to said upper panel (227), in order to form a telescopic upper part (22) of the front wall structure (2), the height of which can be adjusted.

11. Screen according to claim 10, **characterized in that** the movable lower panel (228) is attached to said upper panel (227) by means of an equilibrium system (229), e.g. of the spiral-spring type with constant force (229).

12. Screen according to anyone of claims 6 to 11, **characterized in that** said upper part (22) of the front wall structure (2) comprises a supporting arm (23) pivotally mounted on said corner edge (4) around said pivoting axis (7), and **in that** said at least one panel (227, 228) is movably mounted on said supporting arm (23) for horizontal translational movement.

13. Cover-shaped equipment (40) intended for covering at least part of the height of a radioprotective screen (1) according to claim 12, **characterized in that** it comprises:
- a flexible pocket (401) provided with an opening (401a), said flexible pocket (401) being able to cover at least partially the upper part (22) of the front wall structure (2) by entering the lower edge (224) of the latter into the opening (401a) of said flexible pocket (401), said flexible pocket (401) being provided with at least one transparent part and with means for fixing it to said upper part (22) of the front wall structure (2), and
- at least one flexible panel (402) able to cover at least partially said lateral wall structure (3) and the lower part (21) of said front wall structure (2),
said at least one flexible panel (402) comprising at least one transparent part intended for being positioned in front of the transparent part (381) of said lateral wall structure (3), and fixing means on said lateral (3) and front (2) wall structures, and
- when it comprises a tablet (15) which extends the upper edge (214) of the lower part (21) of the front wall structure, a flexible structure (403) covering said tablet (15) at least partially, either fixed to said panel (402) for covering said lateral wall structure (3) and the lower part (21) of the front wall structure (2), or independent from said panel (402), said flexible structure (403) being provided with means for fixing it to said tablet (15).
